# EUROPÄISCHE PATENTANMELDUNG

(11) **EP 4 388 908 A1**
(43) Veröffentlichungstag der Anmeldung: **26.06.2024**
(21) Anmeldenummer: 23219596.6
(22) Anmeldetag: 22.12.2023
(51) Int. Cl.: A41B 11/00

(54) **KOMPRESSIONSKLEIDUNG AUS FLACHGESTRICK**

(30) Priorität: 22.12.2022 DE 202022107174 U
(71) Anmelder: Kraus, Michael, 90768 Fürth (DE)
(72) Erfinder: Kraus, Michael, 90768 Fürth (DE)
(74) Vertreter: Lösch, Christoph Ludwig Klaus

(57) **Zusammenfassung**

Die Erfindung betrifft eine Kompressionsbekleidung aus Flachgestrick, aufweisend: einem Grundkörper (2) mit einer Längszugkompression und einer Querzugkompression; und mindestens ein, in den Grundkörper eingearbeitetes Elastizitätselement (3, 4) mit einer geringeren Längszugkompression als der das Elastizitätselement (3, 4) umgebende Grundkörper (2) und mit einer Querzugkompression, die der Querzugkompression des das Elastizitätselement (3, 4) umgebenden Grundkörpers (2) entspricht.

## Beschreibung

Die Erfindung betrifft Kompressionsbekleidung aus Flachgestrick.

Kompressionsbekleidungsstücke aus Flachgestrick, beispielweise in Form von Kompressionsstrümpfen oder Armbandagen, sind allgemein bekannt. Ein Flachgestrick besteht im Allgemeinen aus Strickfäden und aus Schussfäden. Der Schussfaden durchdringt die Maschen des Gestricks und ist für die Kompression verantwortlich.

Zur Erfüllung ihres Zweckes - nämlich die Kompression bestimmter Körperregionen - wird Kompressionsbekleidung im Allgemeinen aus einem relativ steifen Flachgestrick hergestellt, wobei aus medizinischer Sicht sichergestellt werden muss, dass sowohl ein ausreichender Ruhedruck als auch ein ausreichender Arbeitsdruck durch die Kompressionsbekleidung aufgebracht wird. Dies führt jedoch zu einem verminderten Tragekomfort, da es - insbesondere in Beuge- oder Streckregionen des Körpers (z.B. Knöchel, Rist,... ) - zu einer eingeschränkten Beweglichkeit und/oder zu Hautirritationen kommen kann.

Aufgabe der Erfindung ist es daher, eine Kompressionsbekleidung mit verbessertem Tragekomfort anzubieten.

Diese Aufgabe wird gelöst durch eine Kompressionsbekleidung mit den Merkmalen des Anspruchs 1. Vorteilhafte Ausführungsformen sind in den Unteransprüchen beschrieben.

Die erfindungsgemäße Kompressionsbekleidung aus Flachgestrick weist einen Grundkörper mit einer Längszugkompression und einer Querzugkompression sowie mindestens ein, in den Grundkörper eingearbeitetes Elastizitätselement auf. Dieses Elastizitätselement besitzt eine geringere Längszugkompression als der das Elastizitätselement umgebende Grundkörper und eine Querzugkompression, die der Querzugkompression des das Elastizitätselement umgebenden Grundkörpers entspricht.

Auf diese Weise besitzt die Kompressionsbekleidung einen oder mehrere Regionen, die durch ihre geringere Längszugkompression im Vergleich zum Grundkörper eine höhere Elastizität bzw. Flexibilität aufweisen, was wiederum eine einfachere Körperbewegung des die Kompressionsbekleidung Tragenden zulässt und den Tragekomfort erhöht.

Unter der Längszugkompression wird hierbei die Kompression der Bekleidung in Richtung des Herzens des die Kompressionsbekleidung Tragenden verstanden. Unter der Querzugkompression wird die Kompression der Bekleidung in orthogonaler Richtung zur Längszugkompression verstanden.

In der erfindungsgemäßen Kompressionsbekleidung aus Flachgestrick ändert sich die Längszugkompression zwischen dem Elastizitätselement und dem umgebenden Grundkörper. Die Querzugkompression hingegen bleibt gleich, denn die Querzugkompression entspricht der Kompressionsklasse der Kompressionsbekleidung und ist die eigentliche Kompression, die für den medizinischen Behandlungserfolg wichtig ist. Diese Querkompression sollte in Richtung Herzen leicht abnehmend sein, um einen Fluss in die Richtung des Herzens zu erreichen. Um diesen Abflussweg zu sichern, ändert sich in den Bereichen des Elastizitätselements die Querzugkompression nicht, wohl aber die Längskompression. Hierdurch erreicht man die erhöhte Flexibilität und die Erhöhung des Tragekomforts.

In vorteilhafter Weise ist das Elastizitätselement in einem Bereich der Kompressionsbekleidung ausgebildet, welches im Tragezustand eine Beuge- oder Streckregion des Körpers des Tragenden kontaktiert. Zu derartigen Beuge- oder Streckregionen gehören insbesondere der Rist-, Knöchel-, Hüft-, Leisten-, Oberarm-, Ellbogen- und/oder Achselbereich.

Das Elastizitätselement kann hierbei derart ausgebildet sein, dass es die Körperregion in Umfangsrichtung vollständig umschließt. Zum Beispiel kann das Elastizitätselement den Knöchelbereich vollständig umgeben. Auf diese Weise kann die Kompression in diesem Bereich zuverlässig beibehalten bleiben.

Es ist jedoch auch möglich, das Elastizitätselement beispielsweise kreis- oder rechteckförmig auf bestimmte, exakt definierte Körperregionen zu begrenzen. Zum Beispiel bietet sich im Ristbereich die Ausbildung eines ovalen oder anderweitig auf die Ristregion begrenzten Elastizitätselements an.

Die Kompressionsbekleidung ist in vorteilhafter Weise als Strumpf oder Strumpfhose, ggf. ohne Fuß oder als Oberkörperbekleidung, insbesondere als Bolero, ausgebildet. Insbesondere kann die Kompressionsbekleidung als kurzer Strumpf bis zum Knie A-D, als langer Strumpf bis Leiste A-G, als Strumpfhose A-T oder als Strumpfhose ohne Fuß B-T ausgebildet sein.

In einer bevorzugten Ausführungsform besteht ein einzelnes Elastizitätselement aus einer Mehrzahl von in räumlicher Nähe zueinander angeordneten Teilelastizitätselementen. Diese Teilelastizitätselemente können sich in ihren Abmessungen und/oder ihrer Längszugkompression unterscheiden. Auf diese Weise kann der Tragekomfort weiter gesteigert werden, da eine noch exaktere Einstellung der Elastizität bzw. Flexibilität in lokal eng definierten Körperregionen möglich ist.

Unabhängig von der Ausbildung eines einzelnen Elastizitätselements als eine Mehrzahl von Teilelastizitätselementen kann die Längszugkompression innerhalb eines einzelnen Elastizitätselements variieren. Insbesondere kann die Längszugkompression innerhalb des Elastizitätselements derart variieren, dass die Längszugkompression in einem Randbereich des Elastizitätselements entweder höher oder niedriger ist als in einem zentralen Bereich des Elastizitätselements. Auf diese Weise kann der Tragekomfort ebenfalls weiter gesteigert werden, da auch hier eine noch exaktere Einstellung der Elastizität bzw. Flexibilität in lokal eng definierten Körperregionen möglich ist.

Erfindungsgemäß weist die Kompressionsbekleidung ein Elastizitätselement auf. Der Tragekomfort wird jedoch weiter gesteigert, wenn die Kompressionsbekleidung mehr als ein einziges Elastizitätselement aufweist. So sind in einem bevorzugten Ausführungsbeispiel zwei Elastizitätselemente vorhanden. Diese Elastizitätselemente sind in bevorzugter Weise in Bereichen der Kompressionsbekleidung ausgebildet, welche im Tragezustand jeweils eine andere Beuge- oder Streckregion des Körpers des Tragenden kontaktieren. Besonders bevorzugt weist das erste Elastizitätselement eine erste Längszugkompression und das zweite Elastizitätselement eine zweite, sich von der ersten Längszugkompression unterscheidenden Längszugkompression auf, wobei sowohl die erste Längszugkompression als auch die zweite Längszugkompression größer sind als die Längszugkompression des Grundkörpers. In ähnlicher Weise sind auch Ausführungsbeispiele denkbar, in denen mehr als zwei Elastizitätselemente mit jeweils sich unterscheidenden Längszugkompressionswerten vorhanden sind. Auch das zweite Elastizitätselement kann als eine Mehrzahl von Teilelastizitätselementen ausgebildet sein und/oder eine Längszugkompression besitzen, die innerhalb des Elastizitätselements variiert.

Die Erfindung ist anhand von Ausführungsbeispielen in den Zeichnungsfiguren weiter erläutert. Es zeigen:
- Fig. 1: ein Ausführungsbeispiel der Kompressionsbekleidung, wobei die Kompressionsbekleidung als Strumpf ausgebildet ist;
- Fig. 2: ein Ausführungsbeispiel der Kompressionsbekleidung, wobei die Kompressionsbekleidung als Strumpfhose mit Fuß ausgebildet ist;
- Fig. 3: ein Ausführungsbeispiel der Kompressionsbekleidung, wobei die Kompressionsbekleidung als weitere Strumpfhose mit Fuß ausgebildet ist;
- Fig. 4: ein Ausführungsbeispiel der Kompressionsbekleidung, wobei die Kompressionsbekleidung als boleroartige Oberkörperbekleidung ausgebildet ist; und
- Fig. 5: ein weiteres Ausführungsbeispiel der Kompressionsbekleidung, wobei die Kompressionsbekleidung als Strumpf ausgebildet ist.

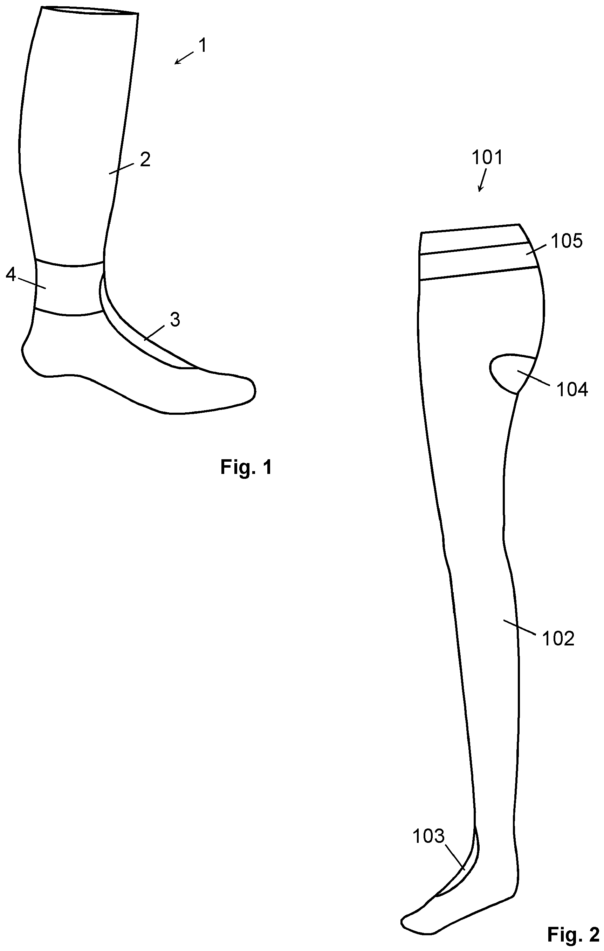

Fig. 1 zeigt einen Strumpf 1. Dieser Strumpf 1 weist einen Strumpfgrundkörper 2, ein erstes Elastizitätselement 3 im Ristbereich und ein zweites Elastizitätselement 4 im Knöchelbereich des Strumpfes 1 auf. Der Strumpf 1 ist als Kompressionsstrumpf aus Flachgestrick ausgebildet und stellt damit eine Kompressionsbekleidung im Sinn der vorliegenden Erfindung dar. Der Strumpfgrundkörper 2, das erste Elastizitätselement 3 und das zweite Elastizitätselement 4 bestehen aus einem Flachgestrick.

Der Strumpfgrundkörper 2 weist eine Längszugkompression in dessen Längsrichtung (d.h. vom Vorderfußbereich in Richtung Beinausschnitt) und, orthogonal zur Längszugkompression, eine Querzugkompression in dessen Umfangsrichtung (d.h. in Umfangsrichtung des Unterschenkels bzw. in Umfangsrichtung des Fußes) auf.

Das erste Elastizitätselement 3 ist im Ristbereich des Strumpfes 1 ausgebildet, genauer von der Mitte des Vorderfußes bis frontal auf Höhe der Knöchel. Das erste Elastizitätselement 3 besitzt eine erste Längszugkompression. Das zweite Elastizitätselement 3 ist in Umfangsrichtung des Unterschenkels um den Knöchel herum ausgebildet. Das zweite Elastizitätselement 4 besitzt eine zweite Längszugkompression. Die erste und zweite Längszugkompression unterscheiden sich voneinander. Jede der ersten und zweiten Längszugkompression ist jedoch höher als die Längszugkompression des Strumpfgrundkörpers 2. Die Querzugkompression des ersten Elastizitätselements 3 und des zweiten Elastizitätselements 4 hingegen entspricht der Querzugkompression des Grundkörpers 2.

Fig. 2 zeigt eine Strumpfhose 101 mit einem Strumpfhosengrundkörper 102, einem ersten Elastizitätselement 103 im Ristbereich, ein zweites Elastizitätselement 104 im Bereich des Gluteus Maximus (genauer im Bereich der Glutealfalte) und ein drittes Elastizitätselement 105 im Taillenbereich der Strumpfhose 101.

Der Strumpfhosengrundkörper 102 besteht aus einem Flachgestrick und die Strumpfhose 101 stellt eine Kompressionsbekleidung im Sinn der vorliegenden Erfindung dar. In Längsrichtung der Strumpfhose 101 (d.h. die Richtung vom Zehenbereich bis zum Taillenbereich) besitzt der Strumpfhosengrundkörper 102 eine Längszugkompression. In Umfangsrichtung jeden Beines bzw. in Umfangsrichtung des Hüftbereichs besitzt der Strumpfhosengrundkörper 102 eine Querzugkompression.

Die Elastizitätselemente 103, 104 und 105 der Strumpfhose 101 bestehen ebenfalls aus Flachgestrick und besitzen jeweils eine Längszugkompression, die höher ist als die Längszugkompression des den jeweiligen Elastizitätselement 103, 104 und 105 umgebenden Bereichs des Strumpfhosengrundkörpers 102.

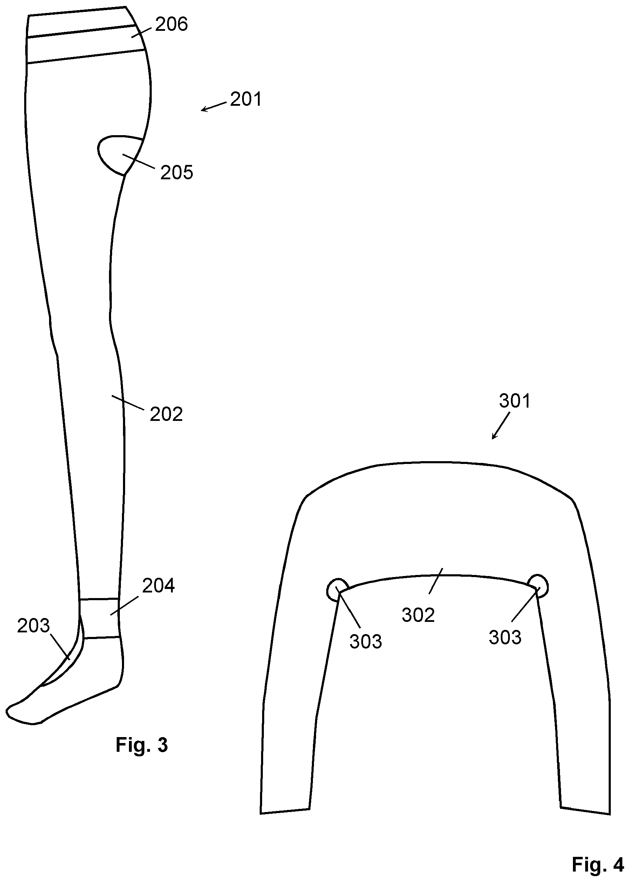

Fig. 3 zeigt eine Strumpfhose 201 (mit Fuß) aus Flachgestrick mit einem Strumpfhosengrundkörper 202, einem ersten Elastizitätselement 203 im Ristbereich, einem zweiten Elastizitätselement 204 im Knöchelbereich, ein drittes Elastizitätselement 205 im Bereich der Glutealfalte und ein viertes Elastizitätselement 206 im Taillenbereich der Strumpfhose 201. Die Strumpfhose 201 stellt eine weitere Kompressionsbekleidung im Sinn der vorliegenden Erfindung dar. In Längsrichtung der Strumpfhose 201 (d.h. die Richtung vom Zehenbereich bis zum Taillenbereich) besitzt der Strumpfhosengrundkörper 202 eine Längszugkompression. In Umfangsrichtung jeden Beines bzw. in Umfangsrichtung des Hüftbereichs besitzt der Strumpfhosengrundkörper 202 eine Querzugkompression. Die Elastizitätselemente 203 bis 206 der Strumpfhose 201 bestehen ebenfalls aus Flachgestrick und besitzen jeweils eine Längszugkompression, die höher ist als die Längszugkompression des den jeweiligen Elastizitätselement 203 bis 206 umgebenden Bereichs des Strumpfhosengrundkörpers 202.

Fig. 4 zeigt eine Rückansicht einer Oberkörperbekleidung 301 aus Flachgestrick in Form eines Boleros mit einem Bolerogrundkörper 302 und zwei Elastizitätselement 303, die deckungsgleich in den beiden Achselbereichen (genauer in den Achselhöhlen) der Oberkörperbekleidung 301 in den Bolerogrundkörper 302 eingearbeitet sind. Die Oberkörperbekleidung 301 stellt eine weitere Kompressionsbekleidung im Sinn der vorliegenden Erfindung dar. Die Elastizitätselemente 303 besitzen jeweils eine Längszugkompression, die höher ist als die Längszugkompression des Bolerogrundkörpers 302.

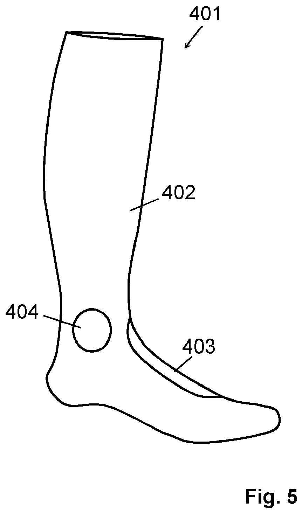

Fig. 5 zeigt einen weiteren Strumpf 401. Dieser Strumpf 401 weist einen Strumpfgrundkörper 402, ein erstes Elastizitätselement 403 im Ristbereich und ein zweites Elastizitätselement 404 im Knöchelbereich des Strumpfes 401 auf. Der Strumpf 401 ist als Kompressionsstrumpf aus Flachgestrick ausgebildet und stellt damit ebenfalls eine Kompressionsbekleidung im Sinn der vorliegenden Erfindung dar. Der Strumpfgrundkörper 402, das erste Elastizitätselement 403 und das zweite Elastizitätselement 404 bestehen aus einem Flachgestrick. Anders als beispielsweise das Elastizitätselement 4 in Fig. 1 oder das Elastizitätselement 204 in Fig. 3 umgibt das Elastizitätselement 404 den Knöchelbereich nicht ringförmig, sondern ist kreisförmig im Bereich des äußeren Knöchels ausgebildet. Die Elastizitätselemente 403 und 404 besitzen jeweils eine Längszugkompression, die höher ist als die Längszugkompression des Strumpfgrundkörpers 402.

### BEZUGSZEICHENLISTE

- 1: Strumpf
- 2: Strumpfgrundkörper
- 3, 4: Elastizitätselement
- 101: Strumpfhose
- 102: Strumpfhosengrundkörper
- 103, 104, 105: Elastizitätselement
- 201: Strumpfhose
- 202: Strumpfhosengrundkörper
- 203, 204, 205, 206: Elastizitätselement
- 301: Oberkörperbekleidung
- 302: Bolerogrundkörper
- 303: Elastizitätselement
- 401: Strumpf
- 402: Grundkörper
- 403, 404: Elastizitätselement

## Patentansprüche

1. Kompressionsbekleidung aus Flachgestrick, aufweisend:
- einem Grundkörper (2) mit einer Längszugkompression und einer Querzugkompression; und
- mindestens ein, in den Grundkörper eingearbeitetes Elastizitätselement (3, 4) mit einer geringeren Längszugkompression als der das Elastizitätselement (3, 4) umgebende Grundkörper (2) und mit einer Querzugkompression, die der Querzugkompression des das Elastizitätselement (3, 4) umgebenden Grundkörpers (2) entspricht.

2. Kompressionsbekleidung nach Anspruch 1, wobei die Kompressionsbekleidung als Strumpf (1, 401) oder Strumpfhose (101, 201) ausgebildet ist und sich das Elastizitätselement im Ristbereich, im Knöchelbereich, im Leistenbereich und/oder im Bereich der Glutealfalte des Strumpfes (1, 401) bzw. der Strumpfhose (101, 201) befindet.

3. Kompressionsbekleidung nach einem der vorhergehenden Ansprüche, wobei die Kompressionsbekleidung als Oberkörperbekleidung (301) ausgebildet ist und sich das Elastizitätselement im Achselbereich Oberkörperbekleidung (301) befindet.

4. Kompressionsbekleidung nach einem der vorhergehenden Ansprüche wobei das Elastizitätselement aus einer Mehrzahl von in räumlicher Nähe zueinander angeordneter Teilelastizitätselementen besteht.

5. Kompressionsbekleidung nach Anspruch 4, wobei die Teilelastizitätselemente unterschiedliche Längszugkompressionswerte und/oder unterschiedliche Abmessungen aufweisen.

6. Kompressionsbekleidung nach einem der vorhergehenden Ansprüche, wobei die Längszugkompression innerhalb des Elastizitätselements variiert.

7. Kompressionsbekleidung nach Anspruch 6, wobei die Längszugkompression innerhalb des Elastizitätselements derart variiert, dass die Längszugkompression in einem Randbereich des Elastizitätselements höher ist als in einem zentralen Bereich des Elastizitätselements.

8. Kompressionsbekleidung nach Anspruch 6, wobei die Längszugkompression innerhalb des Elastizitätselements derart variiert, dass die Längszugkompression in einem Randbereich des Elastizitätselements geringer ist als in einem zentralen Bereich des Elastizitätselements.

9. Kompressionsbekleidung nach einem der vorhergehenden Ansprüche, aufweisend ein erstes Elastizitätselement mit einer ersten Längszugkompression und ein zweites Elastizitätselement mit einer zweiten, sich von der ersten Elastizität unterscheidenden Längszugkompression, wobei sowohl die erste Längszugkompression als auch die zweite Längszugkompression größer sind als die Längszugkompression des Grundkörpers.

10. Kompressionsbekleidung nach einem der vorhergehenden Ansprüche, wobei sich der Grundkörper und das Elastizitätselement beide aus Flachgestrick sind, sich aber in den bei der Herstellung verwendeten Strickparametern und/oder dem verwendeten Garn unterscheiden.
